# EUROPEAN PATENT APPLICATION

(11) **EP 1 875 864 A1**
(43) Date of publication of application: **09.01.2008**
(21) Application number: 06725783.2
(22) Date of filing: 24.02.2006
(51) Int. Cl.: A61B 6/00, G03B 42/02

(54) **SUPPORT FOR RADIOGRAPHIC TELEMETRY RACK**

(30) Priority: 25.04.2005 ES 200501013 U
(71) Applicant: Solaz Novajarque, Rafael, 46017 Valencia (ES); Velasco Martinez, Antonio, 46017 Valencia (ES)
(72) Inventor: Solaz Novajarque, Rafael, 46017 Valencia (ES); Velasco Martinez, Antonio, 46017 Valencia (ES)
(74) Representative: Irache Pereira Tona, Maria
(86) International application number: PCT/ES2006/000085
(87) International publication number: WO 2006/114456

(57) **Abstract**

Support for radiographic telemetry chassis composed by a frame (2) with an opening (4) on one side and an orifice (5) on the opposite side to allow the extraction of the chassis; it also has an opening (6) on the upper side through which the smaller chassis is inserted - chassis being thus anchored to each other - and it is possible to add an anti-diffusing grill (1) to the front of the support to be used for digital radiography, with a locking lid (3) that can be moved to allow different fastening thicknesses.

## Description

### Purpose of the invention

The present invention, as indicated by its title, refers to a support for radiographic telemetry chassis that has been designed to obtain remarkable advantages as compared to other existing devices for similar purposes.

The aim of this invention is to join two chassis together on a support that has a non-diffusion grill and a grid. The support sits on a framework or structure with guides through which the radiographic chassis slide; it has a side opening (right or left) to insert one chassis (measures 35 x 85 cm) and on the opposite side it has an oval opening to allow removing it. The second chassis (measures 35 x 43 cm) is introduced through the upper opening that also has guides, the two chassis being thus joined together inside the support. Covering the entire frontal section, a non-diffusing grill and its corresponding grid can be placed.

On the back section, a lid has been placed so that the structure can be hanged over ONE motorized moving grid, directly over a fixed one, or on a wall.

### Background of the invention

Some supports or methods that join two chassis to perform one radiographic telemetry are known. In this sense, devices based on holes placed on the sides of the chassis where bindings are applied to join the chassis can be mentioned. Once joined, the chassis are placed on a hanger or a stand and are introduced sideways through wide guides until they meet a stop.

This system has the disadvantage that mounting the chassis is hard because, in order to face and close the chassis, they have to be held with one hand while with the other the supports have to be placed inside the holes of the joining system. The system has the added disadvantage of a greater loss of time during the process.

There is also another movable hanger that has a stand with wheels, but the process described for the previous case has to be completed before placing both chassis.

### Description of the invention

The support for the radiographic telemetry chassis object of this invention is intended to improve the problem described in the previous section. To this end, the support presents a new working process that allows taking full spine radiographic plates in a simple and easy manner with no prior preparation required.

To achieve this, the chassis support is designed in such a way that the user only has to place the chassis on the corresponding guides - there is an entry point on the lower side of the support to insert the 35 x 85 cm radiographic chassis and an oval opening on the opposite side to allow removing it. The upper part has the insertion point for the 35 x 43 cm radiographic chassis. The guides used to insert the chassis are adjusted so that both chassis fit seamlessly as if they were only one.

### Description of the drawings

As a complement to the description herein included, and in order to help better understand the characteristics of the invention, a series of descriptive but not limiting figures have been attached to this descriptive summary as an integral part of it, showing the following:
Figure 1.- Perspective view of the pieces constituting the support of the utility model.
Figure 2.- Side view of the support.
Figure 3.- Other side view of the support.
Figure 4.- Upper view of the support.
Figure 5.- Insertion of the lower chassis.
Figure 6.- Insertion of the upper chassis.

### Preferred materialisation of the invention

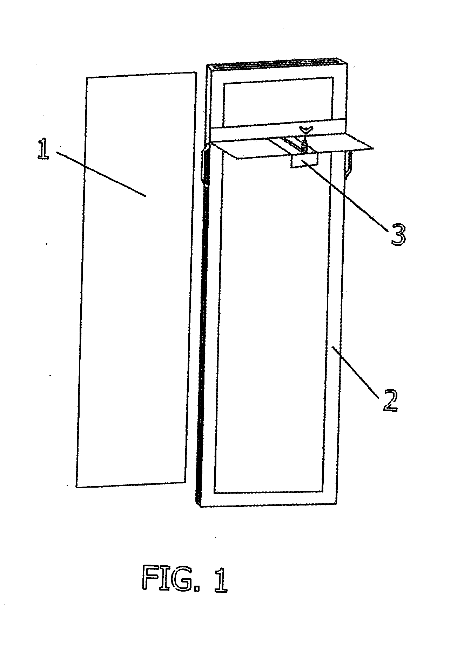

As shown in the figures attached, there is a support (Figure 1) that is composed by a non-diffusing plate or grill (1) whose measures are 38.7 x 126 cm, a frame (2) whose measures are 39 x 126.5 cm, and a locking lid (3) that can be moved to allow different locking thicknesses. The frame has two handles to facilitate its handling and installation.

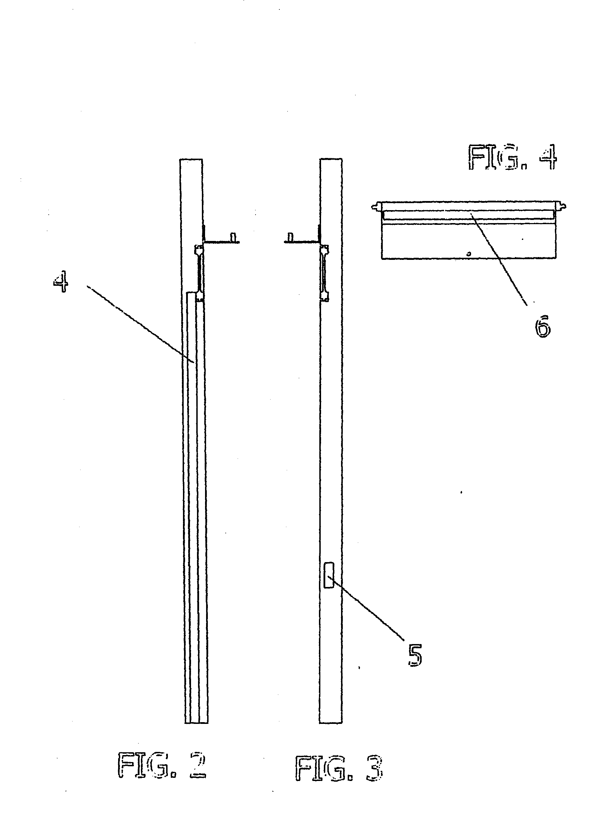

Figure 2 shows the side of the support with the opening for the 35 x 85 cm chassis (4). Figure 3 shows the other side of the support with the 8-cm oval opening (5).

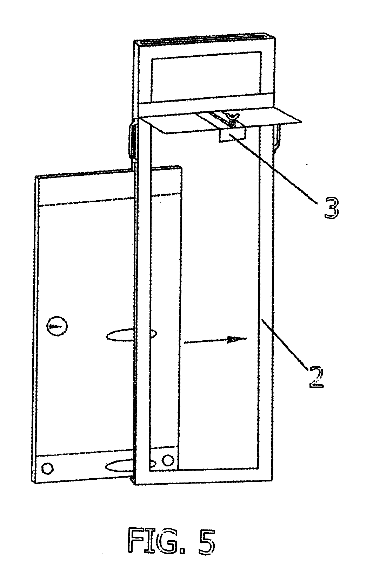

Figure 4 shows the upper view of the support with the opening for the 35 x 43 cm upper chassis (6). Figure 5 shows the movement required to insert the 35 x 85 cm chassis.

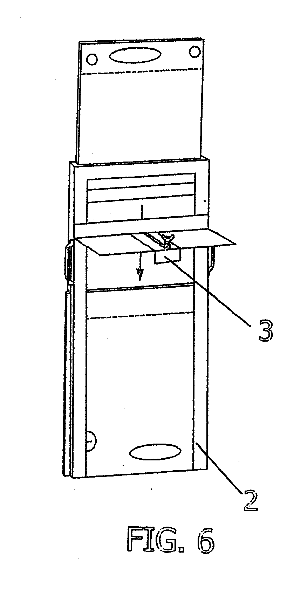

Figure 6 represents the insertion movement for the 35 x 43 cm chassis that slides until meeting the other chassis, the two chassis being thus joined.

After describing in detail the nature of the present invention, as well as a possible practical application, all that remains to be stated is that both its shape and composing materials can be modified provided that such modifications do not substantially affect the characteristics claimed in the following section.

## Claims

1. Support for radiographic telemetry chassis, **characterised by** the presence of a frame (2) with an opening (4) on one side to insert the largest chassis and an orifice (5) on the opposite side to allow the extraction of the chassis; it also has an opening (6) on the upper side through which the smaller chassis is inserted - chassis being thus anchored to each other - and it is possible to add an anti-diffusing grill (1) to the front of the support to be used for digital radiography, with a locking lid (3) that can be moved to allow different fastening thicknesses.

## Amended claims

### Amended claims under Art. 19.1 PCT

**1.** Support for radiographic telemetry chassis, **characterised by** the presence of an only frame (2) with two handles for its manipulation and an opening (4) on the larger side to insert the largest chassis by using guides, and an orifice (5) on the opposite side to allow the extraction of this chassis; it also has an opening (5) on the upper side through which the smaller chassis is guided until both chassis are anchored to each other in a coplanar way. It is possible to add an anti-diffusing grill (1) to the front of the support to be used for digital radiography, with a locking lid (3) that can be moved to allow different fastening thicknesses.
